# EUROPEAN PATENT SPECIFICATION

(11) **EP 1 897 590 B1**
(45) Date of publication and mention of the grant of the patent: **09.11.2016**
(21) Application number: 05822675.4
(22) Date of filing: 12.09.2005
(51) Int. Cl.: A61N 2/00, A61K 51/00

(54) **PREPARATION FOR USE IN A METHOD OF MAGNETIC THERAPY OF MALIGNANT NEOPLASMS**
ZUBEREITUNG ZUR BENUTZUNG IM VERFAHREN EINER MAGNETFELDTHERAPIE VON MALIGNEN NEOPLASMEN
PREPARATION POUR L'UTILISATION DANS LE TRAITEMENT MAGNETIQUE DES NEOPLASIES MALIGNES

(30) Priority: 17.06.2005 RU 2005118803
(43) Date of publication of application: 12.03.2008
(73) Proprietor: Tishin, Aleksandr Mettalinovich, Moscow 141190 (RU)
(72) Inventor: Tishin, Aleksandr Mettalinovich, Moscow 141190 (RU)
(74) Representative: Meissner Bolte Partnerschaft mbB
(86) International application number: PCT/RU2005/000462
(87) International publication number: WO 2006/135270

(56) References cited:
- JP-A- 2005 090 921
- RU-C1- 2 082 458
- US-A- 4 323 056
- US-A- 5 236 410
- US-A1- 2003 028 071
- GLINKA N.L.: 'Obschaya khimiya m., Izdatelstvo "Khimiya"', pages 540 - 541, XP008143758
- ABRAMOVICH A.I. ET AL.: 'Gigantsky magnitokalorichesky effekt ablizi temperatury Kjuri v Sm 0.6 Sr 0.4MnO3 manganite' FIZIKA TVERDOGO TELA vol. 43, no. 4, 2001, pages 687 - 689, XP008122660
- FELIX CASANOVA I FERNANDEZ: 'Magnetocaloric effect in Gd5 (Si x Ge1x)4 alloys' UNIVERSITAT DE BARCELONA December 2003, page 8, AND 13 - 14
- GSCHNEIDNERJR K A ET AL: "Recent developments in magnetocaloric materials; Recent developments in magnetocaloric materials", REPORTS ON PROGRESS IN PHYSICS, INSTITUTE OF PHYSICS PUBLISHING, BRISTOL, GB, vol. 68, no. 6, 1 June 2005 (2005-06-01), pages 1479-1539, XP020084658, ISSN: 0034-4885, DOI: 10.1088/0034-4885/68/6/R04

## Description

### Technical field

The present disclosure relates to magnetotherapy of malignant neoplasm.

### Background art

The current methods for malignant tumors treatment are surgical, chemotherapeutic and radiation. Surgical treatment is the most effective in case of early detection of the tumor. With common types of tumors the surgical treatment is often can't be used or is not applicable.

The most preferred method for conservative malignant tumor treatment is the selective chemotherapy which consists in the injection of antineoplastic agent into blood vessels that supply the tumor. In recent years, malignant tumors have been more frequently treated by intraorganic arterial occlusion which is carried out in combination with chemotherapy or without it.

Recently magnetic field and magnetic materials have been known to gain wide usage in the production of dosage band and pharmaceutical products and also in magnetotherapy for diagnostics and treatment of various diseases.

Methods are known for tumor treatment by the intraorganic arterial occlusion which consists in the injection of the powder iron (reduced, reprecipitated or carbonyl iron) in the form of suspension followed by the location of said suspension in the specific area by means of the external magnetic field, said powder iron is suspended into the physiological solution, polyvinylpyrrolidone solution, albumine solution or other liquid medium.

Such methods allow to occluse great and small vessels thus providing the ischemic necrosis of the tumor affected organ. However sometimes the sure occlusion of the whole organ's vascular system can't be provided using the external magnetic field. The supply of the tumor can be maintained by means of the collateral circulation growth. Due to this such methods are not sufficiently effective, and first of all they are not effective for treatment locally spread tumors.

Methods are known which comprises treating the tumor by the embolization of vessels with silicon compositions, where said silicon compositions solidify into the vascular bed. In this case the need for using external magnetic field is eliminated. However in this case there is a risk of thrombembolia because the embolizate can enter into the general system bloodstream.

A method of selective infarct of a tumor is known according to which a silicon composition comprising carbonyl iron microspheres is injected into the tumor. Due to this the embolization procedure can be carried out into the magnetic field. This allows the newly formed embolus, before they are completely formed, to withstand the arterial blood flow, thus reducing the risk of the entering of said embolus into the general bloodstream and then into the vitally important systems and organs. Moreover, the addition of iron into the composition initiates the local granulomatosis, which results in the better defined fibrotization. Such method, which is used for kidney tumor treatment, allows to occluse the bleeding vessels of antihomatosis pathologies (with providing low risk of peripheral embolization), and also allows to completely occluse the main renal artery as well as renal ramuses. Antiograms show that after a certain time kidneys, that have been occlused, contract and their parenchyma atrophies.

This method is more effective and more reliable than those discussed above. However with a large size of the tumor mass this method can't provide the complete necrocitosis of the tumor cells. Moreover after a certain time the tumor supply can be re-established due to the collateral circulation growth.

Treatment methods are known which involve subjecting the tumors to ferromagnetic embolization followed by electromagnetic hyperthermia. In this case the ischemic necrosis of the tumor affected organ, which is provided by the embolization of said organ's vascular bed, is enhanced by the coagulation necrosis, which is provided by the heating. Obviously in this case the more long-lasting remission can be expected, especially at the early stages of the neoplastic process.

A method for treating kidney malignant tumors is known, according to which the arterial bed of a tumor affected kidney is subjected to chemical embolization by the oily solution of the 100mg of the dioxadet, and then, the main bed of the renal artery is blocked with the metal spiral. According to this method, chemical embolization provides the greater survival percentage than the ordinary embolization. However the tumor degradation results in the progressive intoxication which leads to the patient deterioration. Cellular elements of the tumor enter into the venous bloodstream together with the tumor degradation products. That's why the tumor dissimination followed by the metastasis still can be expected.

US 5,236,410, 1993, discloses another method of tumor treatment. The method consists in selective catheterization of a hepatic artery or renal artery (in case of kidney tumor). A barium or strontium hexaferrite suspension in dioxadete oily solution is injected through a catheter under the X-ray control with simultaneous induction of the external magnetic field in the tumor area. In case of the large size of a tumor mass the arterial bloodflow is then reduced by the metal spiral. After 1 - 3 days the tumor is subjected to the ultrahigh frequency electromagnetic field or to the ultrasound until said tumor is heated up to the 43 - 43,5°C. After the required temperature has been reached the electromagnetic field or ultrasound exposure is kept during 5 - 45 minutes. On the 6 - 7th and 15 - 20th days and also after 3 - 6 months the tumor is subjected to the puncture biopsy. If viable tumor cells are detected then the hyperthermia procedure is repeated.

Because the tumor cells are simultaneously subjected to chemical treatment and hyperthermia action, this method allows to reduce the risk of tumor cells and tumor degradation products entering into the general bloodstream. As result, the organism intoxication and risk of metastasis are also reduced. Embolisate's radiopacity allows monitoring the state of the tumor. The treatment can be repeated if required.

This method is the most effective for tumor cells destruction in comparison with methods discussed above. It is used for the treatment of primary or metastatic liver and kidney tumors.

However this method has some disadvantages. First of all, the magnetic field strength should be very high to keep the magnetically controlled particles in the arterial bed of the affected organ. The high strength of the magnetic field results in the distortion of X-ray image during the angiographic study. The second disadvantage is that the possibility of fat thrombembolia is not eliminated even in case of removing the magnetic field. It relates mainly to cases when the patient conditions don't require arterial bloodstream reduction, and also when there are intraorganic and especially intratumoral venous-arterial shunts. In addition, there is a risk of dioxadete washing away. This results in the reduction of dioxadete concentration into the tumor area and thus leads to the reduction of chemotherapeutical treatment effectiveness. This risk is reduced to a great extend in case of arterial bloodstream reduction which is made when the tumor mass is large sized. If the metal spiral is used for this purpose then during the hyperthermia there is a risk of the strong heating of said metal spiral and of surrounding healthy tissues. On the other hand, the insufficient heating can't provide the complete necrosis of the tumor. The depth of the ultrahigh frequency currents penetration does not exceed 7 centimeters. That's why the presence of viable tumor cells into the large sized tumor mass is associated with the risk of collateral growth in the future. In this case the heating can contribute to the bloodstream establishing and tumor growth.
RU 2065734, 27.08.1996 discloses a method comprising the treatment of tumor by injection of oily solution of a liposoluble antineoplastic agent, by the intraorganic arterial occlusion followed by the local electromagnetic hyperthermia of the tumor. The antineoplastic agent is injected before the occlusion. The occlusion is carried out using the ferrite-and-silicon composition. The hyperthermia is carried out in 3 - 4 weeks after the occlusion by the high frequency currents at 44 - 46°C during 45 - 60 minutes.
The dioxadete in amount of 20 - 100mg which is added to the 5 - 10ml of maiodide or iodinelipol is preferred for use as the antineoplastic agent. The preferred ferritic-and-silicon composition is the carbonyl iron in amount of 2 - 4g, which is suspensed into the 20ml of dimethil-methil-vinyl-polysiloxane and oligo-dimethil-siloxane mixture, whereas the suspension is produced in the presence of chloroplatinic acid and oligo-hydride-siloxane in amount which is enough for the catalytic effect. The composition should be injected in amount which is sufficient for filling the arterial bed of the tumor.

RU 2002133709, 20.06.2004 discloses a method for tumor treatment which includes the use of a therapeutic agent and radiation exposure. The therapeutic agent is prepared by the water electrolysis at 50 - 60°C during 40 - 45 minutes with the DC 0,45 - 0,5mA and 6 - 7V. The water which is full of electrons, ions and hydrated ions of the trace elements is subjected to the constant magnetic field (5000 - 1000 oersted) until its pH reaches 8,4 - 9,4, whereby the percentage ratio of components' masses in electrodes is following
Ag 97,5- 99,5
Cu 1,5 - 0,28
Zn 0,85 - 0,21
Fe 0,15 - 0,1

The above therapeutic agent is ingested in amount of 100 - 150g in the evening, at night and in the morning with the simultaneous radiation exposure of each tumor integumen by the negative charge electrostatic field (0,5-1 cm3/ oersted) during 10 - 15 minutes from the height of about 5 - 10mm. Then the tumor mass is subjected to the negative charges exposure.

The negative charges field exposure on the skin integument of the tumor mass is carried out 3-4 times a day for 10-15 minutes. Though the wide variety of methods for malignant tumor treatment are known, however this problem is still urgent and needs more effective cancer cures. The information is given below which regards to the improved and innovative methods for oncolysis by the magnetic field.

It is known that temperatures under 39°C stimulate tumor growth, at temperatures higher than 39°C the viability of tumor cells is weakened, and temperatures of 40-42°C cause necrocytosis of tumor cells. Once subjected to 42°C for one hour, the tumor tissue undergoes irreversible changes. Non-malignant tissue is still vital after subjection to 43°C for 150 minutes. Even at 46°C non-malignant tissue remains vital for up to one hour. Thus, the damage rate of the tumor depends on the temperature level and exposure time (Kulemin V.V., Kotomin S.V., Albitsky V.B, Varigin Y.A. «Obschaya upravlyaemaya eksogennaya gipertermiya s hypertermiey i chimioterapiey pod narcosom s hypotermiey mozga pri lechenii zlokachestvennykh opukholey. Physiceskaya medicina, 1991, pp. 21- 29»).

A method of hyperthermic electromagnetic therapy of malignant neoplasms is known, wherein the tumor is subjected to the local heating up to 42-44°C by HF, UHF or SHF electromagnetic radiation and then is kept at this temperature for 30 - 120 minutes (the temperature deviation should be ±0,5°C). The temperature of the area being heated is controlled by invasive needle thermocouple sensors having the diameter up to 1 mm, or by invasive catheter thermocouple sensors having the diameter up to 2 mm. The sensors are inserted into the area to be heated (Konoplyanikov A.T. Electromagnitnaya hypertermiya (microwave 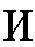 ultra high frequency) pri lecheni opukholevykh i neopukholevykh zabolevaniy. Physical medicine, 1991, pp. 1-11).

A method of oncolysis using magnetic material is known, wherein the said magnetic material consists of a magnetic medium and large-size ferromagnetic particles (5-20 micrometers and more). Said particles are inserted (implanted) into the tumor and then are subjected to the HF field exposure. This provides the heating to sufficient temperature at which the tumor degradates while healthy tissue remains vital, since the necrocytosis of healthy cells does not occur until 43,5°C (US 4,323,056, 1980). The major drawbacks of this method are:
1. Relatively large particles cannot be injected into the dense structure tumor, while the use of small particles is not effective since they are poorly heated in the HF field;
2. The heating of tumor tissue is not uniform, that creates the risk of tumor growth initiation, since at 38-41°C the initiation of tumor cells proliferation occurs.
3. When the temperature reaches the level, which is lethal for tumor cells, the necrocytosis of said tumor cells occurs. This results in the emission of tumor degradation products into the blood-vascular system. In this case, acute intoxication of the organism occurs which sometimes can be fatal.

A method of oncolysis using ferromagnetic material is known. Said ferromagnetic material consists of ultrafine metallic iron particles having the size of 0,2-1,0 micrometers. The method comprises injecting into a tumor a suspension of said ultrafine particles in an antineoplastic agent (8% gelatinome solution), wherein the tumor is then subjected to the HF field exposure (RU 2026083 09/01/1995).

This method allows to occluse large and small blood vessels thus providing the ischemic necrosis of a tumor affected organ. However, this method is not effective enough because it does not provide the total necrosis of tumor cells.

A method for the hyperthermic electromagnetic therapy of malignant neoplasm is known according to which a suspension of fine magnetite particles (which have the size of 20-25 nm) is injected intravenously or intramuscularly into the tumor area. Then the tumor is heated. It shall be noted that the concentration of iron particles after they are injected into the area to be heated is more than 10⁶ particles/cm³ [3] (Matushkin I.F., Tsybukov O.N., et al Ferromagnitnaya UF-hypertermiya subkletochnykh struktur zlokachestvennoy opukholi v experimente, Proceedings of the 2nd All-Russia symposium with international participants. Minsk 1990,, pp.47-48).

In the known method, heating is achieved by using electromagnetic waves of HF band (13,56MHz). Electromagnetic energy is supplied to the area to be heated through whip antennas which are invasively inserted into the said area. High contrast of the area to be heated in terms of the electromagnetic energy absorption coefficient increases the selectivity and efficiency of treatment. As a result, the tumor cells are heated more intensively in comparison with non-malignant cells. However, the above mentioned known methods for hyperthermic elecrtomagnetic therapy have some drawbacks:
- invasive insertion of extraneous bodies, such as whip antennas and temperature sensors, is highly traumatizing to the area or organ to be heated;
- no reliable information about the temperature distribution in the area being heated is available because sensors measure the temperature in their close proximity areas only;
- heating of tissues is not uniform because there are no physical mechanisms which can provide the self-holding temperature equalization through the whole area being heated; the thermal conductivity of tissues could act as said mechanism, but it is obvious that the thermal conductivity can't provide uniform distribution of the temperature and uniform exposure time for all parts of the area being heated, because a sensor which reaches the predetermined temperature first will deactivate the whole heating system thus causing the cooling of area to be heated;
- frequent activation and deactivation of an electromagnetic radiation generator reduces its life, since its life time depends on the number of activations and deactivations rather than on the continuous work time.

The use of HF and SHF electromagnetic radiation has the following disadvantages. There are two ways of heating substances using high frequency alternating magnetic field:
- heating of a conductor by ohmic losses of eddy currents being induced (in case being discussed the high frequency alternative currents are induced into small magnetite particles; said currents induce surface charges on the opposite sides of the particle);
- dielectric heating due to dielectric losses which occur in electromagnetic field absorption.

If HF band electromagnetic field is used, heating by eddy currents is more effective. If SHF band electromagnetic field is used, dielectric heating is more effective due to the skin effect. Thus, in HF hyperthermic therapy, basically, magnetite particles are heated, while the tissue heating achieved via thermal conductivity. In addition, temperature sensors undergo the most strong heating, as they have much greater size than magnetite particles. Therefore, the temperature measurement reliability is intolerable.

A method of carrying out magnetic therapy (hyperthermic electromagnetic therapy) of malignant neoplasms is known, whose technical essence and results provided are the closest to those of the inventive method. In the known method, ferromagnetic (magnetic material) is used, wherein the size of ferromagnetic particles is 2-30 micrometers and its Curie temperature (Curie point) is within the range of 42-45°C. The following substances can be used as the said ferromagnetic (magnetic material):
- iron and nickel alloy (Fe-Ni 30-40, whereas the Curie temperature is within the range of 35-45°C),
- nickel-and-copper based alloy,
- material which exhibits a metal-to-dielectric or metal-to-semiconductor or dielectric-to-metal phase transition, whereas the phase transition temperature is within the range of 42-45°C (such as *VO₂* -based alloy which includes some alloy additives required for the accurate adjustment of the phase transition temperature).

These materials are used for carrying out the hyperthermic electromagnetic therapy of malignant neoplasms. A suspension of said materials particles are injected into a tumor tissue and then are heated by electromagnetic field energy. HF or SHF radiation is used as the electromagnetic radiation. The wattage of SHF radiation generator is 5-100W (RU 2082458, 27.06.1997).

The first and foremost disadvantage of this method is the harmful effect of HF and SHF radiation on a human organism. Though lately the frequency of radiation being used is reduced down to megahertz range and in this case the long-term irradiation doesn't hurt the organism, the method still did not find use for tumor treatment since it does not provide the required effect. The main reason for the method inefficiency is supposedly that hysteresis loop of Fe, Ni and Cu-based alloys is not sufficiently wide and its form does not allow to provide the required heat production during reverse magnetization.

The closest prior art is formed by document US2003/0028071 A1 using preparations responding to alternating magnetic fields for inducing hyperthermia.

### Detailed description of the invention

In view of the above, the object of the present invention is to provide the increase of heat production by using more effective magnetic material and also by using a method which is fundamentally different from those described above and which is based on the so-called magnetocaloric effect (MCE).

The invention is defined in the appended set of claims. Preparation containing magnetic particles is used in a method of magnetic therapy of malignant neoplasms, according to which magnetic particles are injected into a tumor and then are heated by means of a heat which is produced due to MCE, whereas said particles have preferable size of for example 1 nm to 8 microns and consist of a material,
characterized by having high magnetocaloric effect and magnetic phase transition temperature close to the human body temperature. Said material is selected from the group consisting of noble metals, rare-earth metals (elements), their alloys and intermetallic compounds.

Magnetocaloric effect is defined as the heat release or heat absorption in a magnetic substance under exposure to magnetic field. If these processes occur under adiabatic conditions, the temperature of a substance specimen increases or decreases. The MCE was discovered by E. Warburg in 1881.

MCE is based on the ability of any magnetic material to change its temperature and entropy under the magnetic field exposure just as in case of gas or vapour compression (for example in conventional refrigerators).
The magnetic material temperature changes are caused by redistribution of intrinsic energy of the magnetic substance between the magnetic moment system of its atoms and crystal lattice.
The magnetocaloric effect can be used particularly in the magnetic refrigeration technology, for example in systems for conditioning large spaces, in the equipment for food storages and more particularly in the development of industrial and household refrigerating systems. In such magnetic refrigerators, whose function is based on the magnetocaloric refrigeration principle, various magnetic materials can be used as a working medium.

Thermomagnetic properties of magnets are determined by the magnetocaloric effect. The higher the rate of magnetocaloric effect, the more effective heat release or absorption in a magnetic substance under magnetic field exposure is. This provides the enhancement of magnetic materials properties and the enhancement of magnetotherapy efficiency for the treatment of different diseases (in this particular case the enhancement of the efficiency of malignant neoplasms magnetotherapy is achieved).

Examples of materials which are used in the method defined herein include materials with a high magnetocaloric effect and magnetic phase transition temperature close to the human body temperature (from 36°C up to approximately 37°C) are described in detail (A.M. Tishin, Y.I. Spichkin Magnetocaloric effect and its application, Institute of Physics Publishing, Bristol and Philadelphia, 2003, p. 410-411). In particular said materials are
- noble metals (rhodium, palladium, platinum) based alloys,
- rare-earth elements (metals) such as gadolinium Gd (whose Curie temperature is about 295K and whose MCE is ΔT=5,8K for H=2T),
- alloys or intermetallic compounds of rare-earth elements (metals), such as iron-rhodium alloy *Fe*_{0,49}*Rh*_{0,51} (whose antiferromagnetism-ferromagnetism magnetic phase transition temperature is about 310-316K and whose MCE reaches 13K in 2T range),
- gadolinium-silicon *Gd₅Si₄* (whose maximum MCE temperature is ΔT=8,8K for T=336K and H=5T),
- gadolinium-silicon-germanium *Gd*₅*Si*_{2,06}*Ge*_{1,94} (ΔT=8K in 5T range and for T=306K),
- gadolinium-palladium *Gd*₇*Pd*₃ (ΔT=8,5K for T=323K and H=5T),
- manganese-iron-phosphorus-arsenic *MnFeP*_{0,35}*As*_{0,65} (whose maximum MCE point is T=332K),
- manganese-arsenic MnAs (ΔT=13K for T=318K and H=5T)
and others.

The above magnetic material is used in the form of particles which have the size of for example 1,0 nanometer to 8 micrometer. Magnetic measurements show that the magnetic phase transition temperature of rare-earth metal (REM) alloys and compounds depends to a great extend on the concentration of metals and elements being melted together. The variation of a certain element content into the alloy allows to achieve the required magnetocaloric effect and to provide the required magnetic phase transition temperature which is close to the human body temperature. As a rule, the magnetic phase transition occurs into a wide range of magnetic fields whose strength varies from units of kilooersted to 60 kilooersted and more. Magnetic material particles are produced in accordance with various known technologies, for example by plasma method at a temperature of 10000°C in inert atmosphere (such as argon atmosphere) using particles of a certain metal (element) or metals with initial particle size for example of 50-100 micrometers, or a method described in SU 1746162, 07.07.1992.

According to the method disclosed herein, magnetic material particles are injected into a tumor in the form of a suspension or slurry in a pharmaceutically suitable medium such as water, physiological salt solution, polyvinylpyrrolidone, biopolymers, albumine, protein-A.

Magnetic material particles can also be injected together with antineoplastic agent.

### Example embodiment of the present invention

The aforementioned method can be implemented for example in the following way. The magnetic material *Gd*₇*Pd*₃ in the form of 8 or 3 micrometer particles is injected into the tumor tissue and then is exposed to magnetic field, whereas the strength of said field is 50 kilooersted. In the heating step, the magnetic material is magnetized under adiabatic conditions by quick setting of constant magnetic field that results in the temperature increase by the magnitude which is equal to the magnetocaloric effect (ΔT=7K for T=310K). Then, the magnetic field is deactivated that causes adiabatic demagnetization.

The following equipment was used in the present example embodiment:
- a constant magnet (magnetic field),
- a magnetic material in the form of particles,
- auxiliary devices suitable for injection of magnetic material into tumor tissue and further transportation of the magnetic material and its fixation as required.

The magnetic field can also be produced by a superconducting solenoid. The particles are pre-concentrated in a certain area of a tumor by subjecting to magnetic field gradient. Pre-concentrated in the tumor tissue particles of a magnetic material are uniformly heated by exposure to magnetic field. Due to the heat released as result of MCE, the tissues surrounding the particles are heated up to 40-42°C (313-315K), thus causing the tumor cells necrocytosis. Then, the field is deactivated and magnetic material particles are demagnetized (cooled). After the temperature decrease, the particles are heated due to the thermal interchange with the human body and then are magnetized again. Thus, said particles are heated again to the required temperature under exposure to magnetic field and, in turn, heat the tumor affected tissues. Thus, the realization of a method disclosed herein is based on the use of AMRC (active magnetic regenerative cycle), whereas magnetic particles of said materials act not only as a working medium, which provides heating and cooling under magnetization/demagnetization, but also as a heat generator.

### Industrial applicability

The method of magnetotherapy (hyperthermia) disclosed herein promotes (possibly, in combination with pharmaceutical compositions) destruction of tumor cells without damaging healthy cells.

Thus, the aforementioned method provides increased effectiveness of malignant neoplasm theatment.

## Claims

1. A preparation comprising particles, which consist of a material having a magnetocaloric effect and a magnetic phase transition temperature close to the human body temperature, wherein said material is selected from the group consisting of:
- iron-rhodium alloy *Fe*_{0.49}*Rh*_{0,51},
- gadolinium-silicon *Gd₅Si₄,*
- gadolinium-silicon-germanium *Gd*₅*Si*_{2,06}Ge_{1,94},
- gadolinium-palladium *Gd*₇*Pd*₃*,*
- manganese-iron-phosphorus-arsenic *MnFeP*_{0,35}*As*_{0,65},
- manganese-arsenic MnAs,
for use in a method of therapy of malignant neoplasms,
wherein,when the material is magnetized under adiabatic conditions by quick setting of constant magnetic field, its temperature increases due to the magnetocaloric effect, with subsequent release of heat due to adiabatic demagnetization when the magnetic field is deactivated.

2. The preparation according to claim 1, **characterized in that** said material is used in the form of particles having the size from 1 nanometer to 8 micrometer.

3. The preparation according to any one of claims 1 or 2, **characterized in that** said material is used in the form of a suspension in a pharmaceutically acceptable medium.

4. The preparation according to claim 3, **characterized in that** the pharmaceutically acceptable medium is selected from water, physiological salt solution, polyvinylpyrrolidone, biopolymers, albumine, protein-A.

5. The preparation according to any one of claims 1 to 4, **characterized in that** said material is used in combination with antineoplastic agent.

6. The preparation according to any one of claims 1 to 5 for use in a method comprising injection of particles into a tumor tissue followed by heating with electromagnetic field energy, whereby the heating comprises:
a) magnetizing the material in adiabatic conditions by quick setting of a constant magnetic field that results in temperature increase due to the magnetocaloric effect; and then
b) deactivating the magnetic field causing adiabatic demagnetization of the particles allowing them to cool;
whereby steps a) and b) are repeated.

## Patentansprüche

1. Zubereitung, welche Partikel umfasst, die aus einem Material mit einem magnetokalorischen Effekt und einer Übergangstemperatur der Magnetphase nahe an der menschlichen Körpertemperatur bestehen, wobei das Material ausgewählt ist aus der Gruppe bestehend aus:
- Eisen-Rhodium-Legierung Fe_{0,49}Rh_{0,51},
- Gadolinium-Silicium Gd₅Si₄,
- Gadolinium-Silicium-Germanium Gd₅Si_{2,06}Ge_{1,94},
- Gadolinium-Palladium Gd₇Pd₃,
- Mangan-Eisen-Phosphor-Arsen MnFeP_{0,35}As_{0,65},
- Mangan-Arsen MnAs,
zur Verwendung in einem Verfahren zur Therapie von malignen Neoplasmen,
wobei, wenn das Material unter adiabatischen Bedingungen durch rasches Einstellen eines konstanten Magnetfelds magnetisiert wird, seine Temperatur aufgrund des magnetokalorischen Effekts ansteigt und anschließend aufgrund von adiabatischer Demagnetisierung Wärme freigesetzt wird, wenn das Magnetfeld deaktiviert wird.

2. Zubereitung nach Anspruch 1, **dadurch gekennzeichnet, dass** das Material in Form von Partikeln mit einer Größe von 1 Nanometer bis 8 Mikrometern verwendet wird.

3. Zubereitung nach einem der Ansprüche 1 oder 2, **dadurch gekennzeichnet, dass** das Material in Form einer Suspension in einem pharmazeutisch annehmbaren Medium verwendet wird.

4. Zubereitung nach Anspruch 3, **dadurch gekennzeichnet, dass** das pharmazeutisch annehmbare Medium ausgewählt ist aus Wasser, physiologischer Kochsalzlösung, Polyvinylpyrrolidon, Biopolymeren, Albumin, Protein-A.

5. Zubereitung nach einem der Ansprüche 1 oder 4, **dadurch gekennzeichnet, dass** das Material in Kombination mit antineoplastischem Mittel verwendet wird.

6. Zubereitung nach einem der Ansprüche 1 bis 5 zur Verwendung in einem Verfahren, umfassend Injektion von Partikeln in ein Tumorgewebe und anschließendes Erwärmen mit Energie eines elektromagnetischen Feldes, wobei das Erwärmen umfasst:
a) Magnetisieren des Materials unter adiabatischen Bedingungen durch rasches Einstellen eines konstanten Magnetfelds, das aufgrund des magnetokalorischen Effekts zu einem Temperaturanstieg führt, und dann
b) Deaktivieren des Magnetfelds, was adiabatische Demagnetisierung der Partikel herbeiführt, wodurch sie abkühlen können;
wobei die Schritte a) und b) wiederholt werden.

## Revendications

1. Préparation comprenant des particules, qui consistent en un matériau ayant un effet magnétocalorique et une température de transition de phase magnétique proche de la température du corps humain, ledit matériau étant sélectionné dans le groupe consistant en:
- alliage fer-rhodium Fe_{0,49}Rh_{0,51},
- gadolinium-silicium Gd₅Si₄,
- gadolinium-silicium-germanium Gd₅Si_{2,06}Ge_{1,94},
- gadolinium-palladium Gd₇Pd₃,
- manganèse-fer-phosphore-arsenic MnFeP_{0,35}As_{0,65},
- manganèse-arsenic MnAs,
pour utilisation dans une méthode de traitement de néoplasmes malins,
où, lorsque le matériau est magnétisé dans des conditions adiabatiques par mise en place rapide d'un champ magnétique constant, sa température augmente en raison de l'effet magnétocalorique, avec dégagement de chaleur ultérieur en raison de la démagnétisation adiabatique lorsque le champ magnétique est désactivé.

2. Préparation selon la revendication 1, **caractérisée en ce que** ledit matériau est utilisé sous la forme de particules ayant une taille de 1 nanomètre à 8 micromètres.

3. Préparation selon l'une quelconque des revendications 1 ou 2, **caractérisée en ce que** ledit matériau est utilisé sous la forme d'une suspension dans un milieu pharmaceutiquement acceptable.

4. Préparation selon la revendication 3, **caractérisée en ce que** le milieu pharmaceutiquement acceptable est sélectionné parmi l'eau, une solution saline physiologique, la polyvinylpyrrolidone, les biopolymères, l'albumine, la protéine A.

5. Préparation selon l'une quelconque des revendications 1 à 4, **caractérisée en ce que** ledit matériau est utilisé en association avec un agent antinéoplasique.

6. Préparation selon l'une quelconque des revendications 1 à 5 pour utilisation dans une méthode comprenant
l'injection de particules dans un tissu tumoral suivie par un chauffage avec l'énergie d'un champ électromagnétique, où le chauffage comprend:
a) la magnétisation du matériau dans des conditions adiabatiques par mise en place rapide d'un champ magnétique constant qui aboutit à une augmentation de température en raison de l'effet magnétocalorique ; et ensuite
b) la désactivation du champ magnétique provoquant une démagnétisation adiabatique des particules leur permettant de refroidir;
où les étapes a) et b) sont répétées.
